# EUROPÄISCHE PATENTSCHRIFT

(11) **EP 0 817 790 B1**
(45) Veröffentlichungstag und Bekanntmachung des Hinweises auf die Patenterteilung: **14.11.2001**
(21) Anmeldenummer: 96904793.5
(22) Anmeldetag: 15.02.1996
(51) Int. Cl.: C07H 19/10, C07H 19/207, A61K 31/70

(54) **SPEZIFISCHE LIPIDKONJUGATE VON NUCLEOSID-DIPHOSPHATEN UND DEREN VERWENDUNG ALS ARZNEIMITTEL**
SPECIFIC LIPID CONJUGATES OF NUCLEOSIDE DIPHOSPHATES AND THEIR USE AS DRUGS
CONJUGUES LIPIDIQUES SPECIFIQUES DE DIPHOSPHATES NUCLEOSIDIQUES ET LEUR UTILISATION COMME MEDICAMENTS

(30) Priorität: 16.02.1995 DE 19505168
(43) Veröffentlichungstag der Anmeldung: 14.01.1998
(73) Patentinhaber: Heidelberg Pharma Holding GmbH, 69126 Heidelberg (DE)
(72) Erfinder: ZILCH, Harald, D-68305 Mannheim (DE); HERRMANN, Dieter, D-69115 Heidelberg (DE)
(74) Vertreter: Patentanwälte Zellentin & Partner
(86) Internationale Anmeldenummer: EP9600653
(87) Internationale Veröffentlichungsnummer: WO9625421

(56) Entgegenhaltungen:
- WO-A-91/19726
- DE-A- 4 026 265
- JOURNAL OF MEDICINAL CHEMISTRY, Bd. 33, Nr. 5, 1990, WASHINGTON US, Seiten 1380-1386, XP002005552 HONG C I ET AL: "Nucleoside conjugates. 11. Synthesis and antitumor activity of 1-.beta.-D-arabinofuranosylcytosine and cytidine conjugates of thioether lipids"
- JOURNAL OF MEDICINAL CHEMISTRY, Bd. 34, Nr. 4, 1991, WASHINGTON US, Seiten 1408-1414, XP002005553 PIANTADOSI C ET AL: "Synthesis and evaluation of novel ether lipid nucleoside conjugates for anti-HIV-1 activity"

## Beschreibung

Gegenstand der vorliegenden Erfindung sind neue Phospholipid-Derivate von Nucleosiden der allgemeinen Formel I, 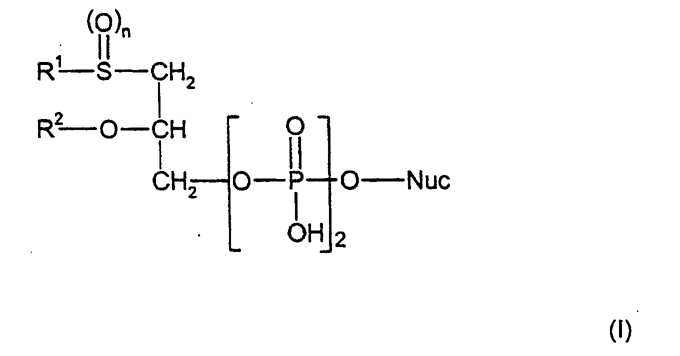 in der
- R¹: einen geradkettig verzweigten, gesättigten oder ungesättigten aliphatischen Rest mit 9 - 14 Kohlenstoffatomen darstellt,
- R²: einen geradkettigen oder verzweigten, gesättigten oder ungesättigten aliphatischen Rest mit 8 - 12 Kohlenstoffatomen darstellt,
- n: 0, 1 oder 2 ist,

Nuc ein Nucleosid oder ein Nucleosid-Derivat aus der Gruppe
-2',3'-Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2'-3'-didehydro-N⁶-(o-methylbenzyl)adenosin
-2',3'-Didesoxy-2',3'-didehydro-N⁶-(2-methylpropyl)adenosin
-2',3'-Didesoxy-3'-azidoguanosin
-3'-Desoxy-3'-azido-thymidin
-2',3'-Didesoxy-3'-fluor-S-chloruridin
-3'-Desoxy-3'-fluorthymidin
-2',3'-Didesoxy-3'-fluoradenosin
-2',3'-Didesoxy-3'-fluor-2,6-diaminopurinribosid
-2',3'-Didesoxy-2'-3'-didehydrocytidin
-3'-Desoxy-2'-3'-didehydrothymidin
-5-Fluoruridin
-6-Mercaptopurin-9-β-D-ribofuranosid
-Acyclovir
-Ganciclovir
-Adenin-9-β-D-arabinofuranosid
-2-Chlor-2'-desoxyadenosin
-3-(2-Desoxy-ß-D-erythro-pentofuranosyl)-3,6,7,8-tetrahydro-imidazo[4,5-d] [1,3] diazepin-8-ol
-Cytosin-9-β-D-arabinofuranosid
-Guanin-9-β-D-arabinofuranosid
-Hypoxanthin-9-β-D-arabinofuranosid
-2'-Desoxy-2-fluoradenosin
-2-Fluoradenin-9-β-D-arabinofuranosid
-2-Fluoradenosin
-2-Amino-6-mercaptopurin-9-ß-D-ribofuranosid
- 6-Methylmercaptopurin-9-β-D-ribofuranosid
-3'-Desoxy-5-fluoruridin
-2-Chloradenosin
-3'-Desoxy-3'-fluoradenosin
-3'-Desoxy-3'-fluorguanosin
-1-(β-D-Arabinofuranosyl)-5-ethinyluracil
-1-(β-D-Arabinofuranosyl)-5-prop-1-inyluracil
-1-(β-D-Arabinofuranosyl)-5-prop-2-inyluracil ist,
sowie deren Tautomere und deren physiologisch verträgliche Salze anorganischer und organischer Säuren bzw. Basen, und diese Verbindungen enthaltende Arzneimittel.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

In der Literatur sind zahlreiche Nucleosiddiphosphatdiacylglycerole und deren Herstellung beschrieben. In Biochim. Biophys. Acta 1165, 45 (1992) und J. Lipid Res. 33, 1211 (1992) ist die Synthese entsprechender Derivate des AZT, DDC und d2T beschrieben.

In Biochim. Biophys. Acta 1084, 307 (1991) und 1086, 99 (1991) ist die Freisetzung des Nucleosidmonophosphats durch mitochondriale Enzymaktivität aus Rattenleber aufgezeigt.

Den Protein-induzierten Intermembrantransfer von antiviralen Derivaten sowie die Synthese beschreibt Biochemistry 31, 5912 (1992) und J. Biol. Chem. 265, 6112 (1990).

Die antitumorale Wirkung von ara-C-diphosphat-Derivaten mit Ether- und Thioetherlipiden in SN1-Stellung ist in LIPIDS 26, 1437 (1991), Drugs of the Future 15, 245 (1990), Exp. Hematol. 17, 364 (1989), J. Med. Chem. 33, 1380 (1990) und Cancer Res. 50, 4401 (1990) beschrieben

EP 0 376 518 zeigt antineoplastische Eigenschaften von 2'-Desoxy-2', 2'-difluornucleosid-Derivaten auf und J. Med. Chem. 25, 1322 (1982) sowie 31, 1793 (1988) gibt Informationen zu Synthese und antitumoraler Wirkung von ara-C-5'-diphosphatdiacylglycerolen.

Die Anmeldungen DD-290-197 und EP 0 432 183 beschreiben die Synthese von Cytidin-5'-diphosphat-1-0-alkylglycerol mit antitumoraler Wirkung und EP 0 355 016 die Synthese von entsprechenden Diphosphatglycerolen.

In DE 35 43 346 ist die Herstellung von ara-C-5'-diphosphat-1-O-octadecyl-2-O-palmitoylglycerin beschrieben und d2T-5'-diphosphat-dimyristoylglycerol mit seiner antiviralen Wirkung ist aus Antimicrob. Agent. Chemother. 36, 2025 (1992) bekannt.

Ein Teil der in dieser Anmeldung beschriebenen Derivate sind von den Patentschriften WO 91/19726 (PCT/US91/04289) und US 4,622,392 umfaßt, jedoch ist die Beschreibung sehr spekulativ und enthält keine konkreten Angaben zur Herstellung der in dieser Anmeldung beschriebenen Verbindungen. Außerdem weisen die in dieser Anmeldung beschriebenen Verbindungen überraschenderweise vorteilhafte Eigenschaften hinsichtlich ihrer pharmakogischen Wirkung auf, die sie von den in den oben genannten Patentschriften beschriebenen Verbindungen unterscheiden.

Die Verbindungen der vorliegenden Erfindung sind neu und weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und 2, verursacht werden.

Besonders geeignet erscheinen die Verbindungen der Formel I zur Behandlung der klinischen Manifestationen der retroviralen HIV-Infektion beim Menschen, wie der anhaltenden generalisierten Lymphadenopathie (PGL), dem fortgeschrittenen Stadium des AIDS-verwandten Komplex (ARC) und dem klinischen Vollbild von AIDS.

Außerdem eignen sich die Verbindungen der Formel I zur Therapie und Prophylaxe von bösartigen Geschwülsten, wie Malignomen und Neoplasien (Karzinomen, Sarkomen, Leukämien, etc.) in der Tumortherapie, sowie zur Hemmung onkogener Viren.

Allgemein sind Verbindungen der Formel I dann von Interesse, wenn das gekoppelte Nucleosid (Nuc) beispielsweise eine zytotoxische, antitumorale, antivirale, antiretrovirale, immunsupprimierende oder immunstimulierende Wirkung aufweist und als Arzneimittel wegen Nebenwirkungen, einer zu geringen therapeutischen Breite oder Organtoxizitäten nicht oder nur eingeschränkt verwendbar ist.

Im Vergleich zu den bisher zur Behandlung eingesetzten Nucleosiden besitzen die erfindungsgemäßen Verbindungen eine geringe Toxizität. Sie besitzen dadurch den Vorteil, daß die Verabreichung von Arzneimitteln, die diese Verbindungen enthalten, über einen längeren Zeitraum hinweg kontinuierlich durchgeführt werden kann und die Absetzung des Präparats oder eine intermittierende Verabreichung wegen unerwünschter Nebeneffekte vermieden werden kann.

Die Verbindungen der vorliegenden Erfindung und ihre pharmazeutischen Zubereitung können auch in Kombination mit anderen Arzneimitteln zur Behandlung und Prophylaxe von verschiedenen Erkrankungen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind bzw. Arzneimittel mit zytostatischer/zytotoxischer oder immunsupprimierender/-stimulierender Wirkung.

Die Konjugate der Formel I zeigen entscheidende Vorteile im Vergleich zum nicht konjugierten Wirkstoff.

Der spezifische, kovalent an den Wirkstoff gebundene Carrier verbessert die Bioverfügbarkeit von schlecht resorbierten Wirkstoffen, die Verträglichkeit von potentiell toxischen Wirkmolekülen und die Verweilzeit von schnell eliminierten oder metabolisierten Arzneimitteln.

Der Carrierteil mit seiner lecithinartigen Struktur, die für den beanspruchten Effekt essentiell ist, verbessert die Penetration/Membrangängigkeit des Wirkstoffs und zeigt in vielen Fällen einen Depoteffekt.

Die gastrointestinale Verträglichkeit der beanspruchten Lipidkonjugate ist vielfach besser als die der reinen Wirkstoffe.

Die Heteroatome in den Resten -S(O)ₙ-R¹ und -O-R² im Lipidteil können nicht durch die aus Lecithin bekannten Carbonsäureester ersetzt werden, da sonst schon im Serum eine hydrolytische Spaltung zu den entsprechenden Lysolecithin-Derivaten/Glycerolestem mit entsprechend schnellerer Elimination des Wirkstoffs erfolgen würde.

Die Thioether/Etherlipide dieser Anmeldung zeigen diese Spaltung im Serum des Menschen nicht.

Auch bei der Resorption zeigt das Lipid-Konjugat eine bessere Penetration durch Membranstrukturen und somit eine bessere Überwindung der Resorptionsbarrieren und z.B. der Blut-Him-Schranke durch erleichterte Diffusion oder evtl. aktiven Transport.

Durch eine bessere Bindung des Konjugats an Plasma- und Gewebeproteine wird ferner die Verteilung in vivo verbessert. Durch normale Biotransformation wird das Konjugat primär vom Thioether zum Sulfoxid oxidiert, was aber aufgrund der fast equipotenten Wirkung des Sulfoxids im Vergleich zum Thioether keinen Nachteil darstellt.

Durch eine langsame Freisetzung des Wirkstoffs aus dem Konjugat wird ein niedriger, aber über einen längeren Zeitraum konstanter Wirkstoffspiegel gewährleistet und ein toxischer Nebeneffekt vermieden.

Der freigesetzte Wirkstoff kann beispielsweise eine zytotoxische, antitumorale, antivirale, antiretrovirale, immunsupprimierende oder immunstimulierende Wirkung aufweisen.

Als mögliche Salze der Verbindungen der allgemeinen Formel I kommen vor allem Alkali-, Erdalkali- und Ammoniumsalze der Phosphatgruppe in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

Die Verbindungen der allgemeinen Formel I können basische Gruppen, insbesondere Amino-Gruppen enthalten, die mit geeigneten Säuren in Säureadditionssalze überführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

Ein aliphatischer Rest in der Definition von R¹ und R² bedeutet eine Alkyl-, Alkenyl- oder Alkinylgruppe.

In der allgemeinen Formel I bedeutet R¹ vorzugsweise eine geradkettige C₁₀-C₁₄-Alkylgruppe. R¹ stellt insbesondere eine Decyl-, Undecyl-, Dodecyl-, Tridecyl- oder Tetradecylgruppe dar.

R² bedeutet vorzugsweise eine geradkettige C₁₀-C₁₂-Alkylgruppe.
R² stellt insbesondere eine Decyl-, Undecyl- oder Dodecylgruppe dar.

Der Rest Nuc steht für ein Nucleosid-Derivat, das über die 5'-Position an die Pyrophosphorsäure des lipophilen Teils der Formel I gebunden ist. Diese Nucleosid-Derivate sind Reste der folgenden Verbindungen:
-2',3'-Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2'-3'-didehydro-N⁶-(o-methylbenzyl)adenosin
-2',3'-Didesoxy-2'-3'-didehydro-N⁶-(2-methylpropyl)adenosin
-2',3'-Didesoxy-3'-azidoguanosin
-3'-Desoxy-3'-azido-thymidin
-2',3'-Didesoxy-3'-fluor-5-chloruridin
-3'-Desoxy-3'-fluorthymidin
-2',3'-Didesoxy-3'-fluoradenosin
-2',3'-Didesoxy-3'-fluor-2,6-diaminopurinribosid
-2',3'-Didesoxy-2'-3'-didehydrocytidin
-3'-Desoxy-2'-3'-didehydrothymidin
-5-Fluoruridin
-6-Mercaptopurin-9-β-D-ribofuranosid
-Acyclovir
-Ganciclovir
-Adenin-9-β-D-arabinofuranosid
-2-Chlor-2'-desoxyadenosin
-3-(2-Desoxy-β-D-erythro-pentofuranosyl)-3,6,7,8-tetrahydro-imidazo [4,5-d] [1,3] diazepin-8-ol
-Cytosin-9-β-D-arabinofuranosid
-Guanin-9-β-D-arabinofuranosid
-Hypoxanthin-9-β-D-arabinofuranosid
-2'-Desoxy-2-fluoradenosin
-2-Fluoradenin-9-β-D-arabinofuranosid
-2-Fluoradenosin
-2-Amino-6-mercaptopurin-9-β-D-ribofuranosid
-6-Methylmercaptopurin-9-β-D-ribofuranosid
-3'-Desoxy-5-fluoruridin
-2-Chloradenosin
-3'-Desoxy-3'-fluoradenosin
-3'-Desoxy-3'-fluorguanosin
-1-(β-D-Arabinofuranosyl)-5-ethinyluracil
-1-(β-D-Arabinofuranosyl)-5-prop-1-inyluracil
-1-(β-D-Arabinofuranosyl)-5-prop-2-inyluracil

Die Verbindungen der allgemeinen Formel I können nach bekannten und publizierten Verfahren hergestellt werden, in dem man z.B.
eine Verbindung der allgemeinen Formel V, 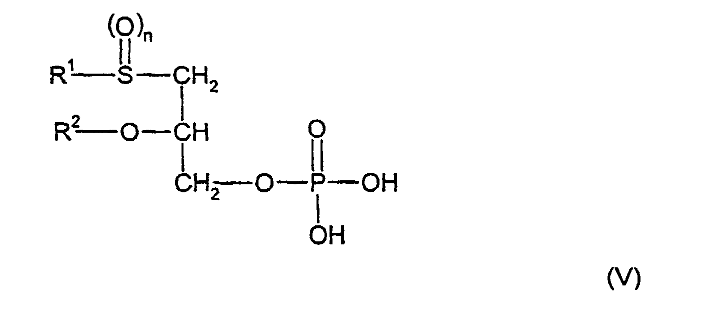 in der R¹, R² und n die angegebenen Bedeutungen besitzen, als Morpholidat mit einer Verbindung der allgemeinen Formel VI, 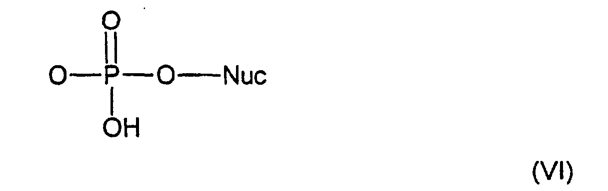 in der Nuc die oben angegebene Bedeutung besitzt,

in Gegenwart einer tert. Stickstoffbase, z.B. Pyridin oder Lutidin, in einem inerten Lösungsmittel, wie z.B. Toluol, oder direkt in Pyridin zur Reaktion bringt und nach erfolgter Hydrolyse gegebenenfalls entsprechend den in der Nucleosidchemie üblichen Verfahren die Sauerstoffschutzgruppen abspaltet, wie dies beispielsweise in J. Lipid Res. 33, 1211 (1992) beschrieben ist.

Alternativ kann auch das Morpholidat der Verbindung der allgemeinen Formel VI mit dem Phosphat der Formel V umgesetzt werden.

Die Herstellung der Verbindungen der allgemeinen Formel V ist in DE 39 29 217 bzw. WO91/05558 beschrieben.

Der allgemeinen Formel I ähnliche Verbindungen sind beschrieben in EP-A-0350287. Dort sind die entsprechenden 1,2-Diester des Glycerins beschrieben.

Die Arzneimittel enthaltend Verbindungen der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées. Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage:
1. (2',3'-Didesoxy-3'-fluor-5-chloruridin)-5'-diphosphorsäure-(3-dodecylmercapto-2-decyloxy)-1 -propylester
2. (3'-Desoxy-3'-azido-thymidin)-5'-diphosphorsäure-(3-dodecylsulfinyl-2-decyloxy)-1-propylester
3. (3'-Desoxy-3'-azido-thymidin)-5'-diphosphorsäure-(3-dodecylsulfonyl-2-decyloxy)-1-propylester
4. (2',3'-Didesoxycytidin)-5'-diphosphorsäure-(3-dodecylmercapto-2-decyloxy)-1-propylester
5. (2',3'-Didesoxyinosin)-5'-diphosphorsäure-(3-dodecylmercapto-2-decyloxy)-1-propylester
7. (6-Mercaptopurin-9-ß-D-ribofuranosid)-5'-diphosphorsäure-(3-dodecylmercapto-2-decyloxy)-1-propylester
8. (2-Chlor-2'-desoxyadenosin)-5'-diphosphorsäure-(3-dodecylmercapto-2-decylmercapto)-1-propylester
9. (3'-Desoxy-2',3'-didehydrothymidin)-5'-diphosphorsäure-(3-dodecylmercapto-2-decyloxy)-1 -propylester
10. (3'-Desoxy-3'-fluorthymidin)-5'-diphosphorsäure-(3-dodecylsulfinyl-2-decyloxy)-1-propylester
11. (2-Fluoradenin-9-β-D-arabinofuranosid)-5'-diphosphorsäure-(3-dodecylmercapto-2-decyloxy)-1-propylester
12. (2-Fluoradenosin)-5'-dlphosphorsäure-(3-dodecylmercapto-2-decyloxy)-1-propylester
13: (2-Amino-6-mercaptopurin-9-β-D-ribofuranosid)-5'-diphosphorsäure-(3-dodecylmercapto-2-decyloxy)-1-propylester
14. (2-Chloradenosin)-5'-diphosphorsäure-(3-dodecylmercapto-2-decyloxy)-1-propylester
15. (Cytosin-1-ß-D-arabinofuranosid)-5'-diphosphorsäure-(3-decylmercapto-2-dodecyloxy)-1-propylester
17. (Adenin-9-ß-D-arabinofuranosid)-5'-diphosphorsäure-(3-decylsulfonyl-2-dodecyloxy)-1-propylester
18. (Guanin-9-β-D-arabinofuranosid)-5'-diphosphorsäure-(3-decylmercapto-2-decyloxy)-1-propylester
19. [3-(2-Desoxy-ß-D-erythro-pentofuranosyl)-3,6,7,8-tetrahydroimidazol [4,5-d] [1,3] diazepin-8-ol]-5'-diphosphorsäure-(3-dodecylmercapto-2-dodecyloxy)-1-propylester
20. (5-Fluoruridin)-5'-diphosphorsäure-(3-undecylmercapto-2-undecyloxy)-1-propylester
21. (2-Chlor-2'-desoxyadenosin)-5'-diphosphorsäure-(3-undecylmercapto-2-undecyloxy)-1-propylester
22. (6-Mercaptopurin-9-β-D-ribofuranosid)-5'-diphosphorsäure-(3-tridecylmercapto-2-decyloxy)-1-propylester
23. (3'-Desoxy-3'-fluoradenosin)-5'-diphosphorsäure-(3-dodecylmercapto-2-nonyloxy)-1-propylester

### Beispiel 1

### (3'-Azido-3'-desoxythymidin)-5'-diphosphorsäure-(3-dodecylmercapto-2-decyloxy)-1-propylester

Analog zu J. Lipid Res. 33, 1211 (1992) wurde die genannte Verbindung hergestellt, indem man eine Lösung von 9.93 g (20 mmol) Phosphorsäuremono (3-dodecylmercapto-2-decyloxy)-1-propylester in 250 ml CH₂Cl₂ unter Rühren bei RT zu 7.0 ml (80 mmol) Morpholin in 300 ml t -Butanol/1,5 ml Wasser tropft und zum Rückfluß erhitzt. Über 2 h wird dann eine Lösung aus 16.5 g (80 mmol) DCC in 400 ml t -Butanol zugegeben, weitere 6 h unter Rückfluß erhitzt und nach dem Abkühlen im Rotationsverdampfer eingeengt. Der Rückstand wird in 800 ml Wasser suspensiert und 3 x mit 300 ml t -Butylmethylether extrahiert. Die vereinigten organischen Phasen werden eingedampft, mehrfach mit Pyridin trockendestilliert und der Rückstand ohne weitere Reinigung in die Folgereaktion eingesetzt.

Durch Umsetzung von 5.35 g (20 mmol) AZT mit POCl₃ in Gegenwart von Proton Sponge in Trimethylphosphat, Hydrolyse mit 1 M Triethylammoniumbicarbonat-Lösung, Eindampfen zur Trockene und Chromatographie des Rückstands an RP 18 mit MeOH/H₂O 5/1 als Eluens erhält man AZT-Monophosphat.

Das rohe Morpholidat und das AZT-Monophosphat werden jeweils in 200 ml Pyridin gelöst, die Lösungen vereinigt und zur Trockene eingedampft. Der Rückstand wird danach in 400 ml abs. Pyridin gelöst und unter N2-Atmosphäre 20 h bei 30°C gerührt.

Nach Entfemen des Lösungsmittels und Ausrühren mit t-Butylmethylether wird der Eindampftrückstand durch präp. Säulenchromatographie an RP 18 mit Methanol/0.01 M Acetat-Puffer 87/13 pH 5 als Eluens gereinigt. Ausbeute 8.8 g (59 % d. Th.). Paste R_{f} = 0,45 (Methanol/H₂O 85/15), RP 18-DC-Platten Merck 15685; R_{f} = 0.32 (Dichlormethan/Methanol/Wasser 65/25/4 und R_{f} = 0.13 (i-Propanol/Butylacetat/Eisessig/Wasser 5/3/1/1) auf die DC-Platten Merck 5719. Die Verbindung kann nach Lösen in Wasser mit konz. Ammoniak als Ammoniumsalz gefüllt werden. Schmp. 79-85°C.

### Beispiel 2

### (3'-Desoxy-3'-fluorthymidin)-5'-diphosphorsäure-mono-(3-dodecylmercapto-2-decyloxy)-1-propylester

Die Verbindung wurde analog zu Beispiel 1 aus dem beschriebenen Morpholidat-Rohprodukt und chromatographisch gereinigtem 3'-Desoxy-3'-fluorthymidin-Monophosphat hergestellt. Ausbeute 47 % d. Th., Paste R_{f} = 0.42 (Methanol/H₂O 85/15), RP 18-DC-Platten Merck.

## Patentansprüche

1. Phospholipid-Derivate von Nucleosiden der allgemeinen Formel I, 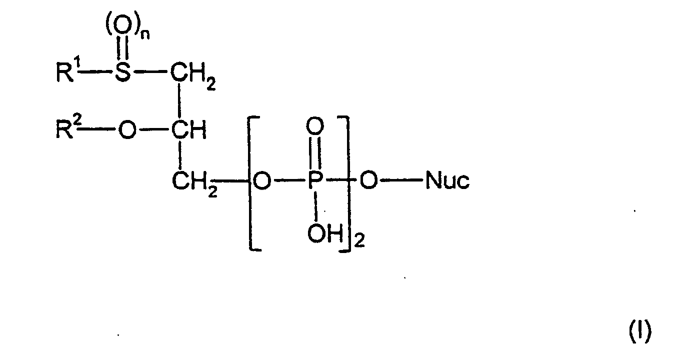 in der
R¹ einen geradkettig verzweigten, gesättigten oder ungesättigten aliphatischen Rest mit 9 - 14 Kohlenstoffatomen darstellt,
R² einen geradkettigen oder verzweigten, gesättigten oder ungesättigten aliphatischen Rest mit 8 - 12 Kohlenstoffatomen darstellt,
n 0, 1 oder 2 ist,
Nuc ein Nucleosid oder ein Nucleosid-Derivat aus der Gruppe
-2',3'-Didesoxy-3'-azidouridin
-2',3'-Didesoxyinosin
-2',3'-Didesoxyguanosin
-2',3'-Didesoxycytidin
-2',3'-Didesoxyadenosin
-3'-Desoxythymidin
-2',3'-Didesoxy-2'-3'-didehydro-N⁶-(o-methylbenzyl)adenosin
-2',3'-Didesoxy-2',3'-didehydro-N⁶-(2-methylpropyl)adenosin
-2',3'-Didesoxy-3'-azidoguanosin
-3'-Desoxy-3'-azido-thymidin
-2',3'-Didesoxy-3'-fluor-S-chloruridin
-3'-Desoxy-3'-fluorthymidin
-2',3'-Didesoxy-3'-fluoradenosin
-2',3'-Didesoxy-3'-fluor-2,6-diaminopurinribosid
-2',3'-Didesoxy-2'-3'-didehydrocytidin
-3'-Desoxy-2'-3'-didehydrothymidin
-5-Fluoruridin
-6-Mercaptopurin-9-β-D-ribofuranosid
-Acyclovir
-Ganciclovir
-Adenin-9-β-D-arabinofuranosid
-2-Chlor-2'-desoxyadenosin
-3-(2-Desoxy-β-D-erythro-pentofuranosyl)-3,6,7,8-tetrahydro-imidazo [4,5-d] [1,3] diazepin-8-ol
-Cytosin-9-β-D-arabinofuranosid
-Guanin-9-β-D-arabinofuranosid
-Hypoxanthin-9-β-D-arabinofuranosid
-2'-Desoxy-2-fluoradenosin
-2-Fluoradenin-9-β-D-arabinofuranosid
-2-Fluoradenosin
-2-Amino-6-mercaptopurin-9-β-D-ribofuranosid
-6-Methylmercaptopurin-9-β-D-ribofuranosid
-3'-Desoxy-5-fluoruridin
-2-Chloradenosin
-3'-Desoxy-3'-fluoradenosin
-3'-Desoxy-3'-fluorguanosin
-1-(β-D-Arabinofuranosyl)-5-ethinyluracil
-1-(β-D-Arabinofuranosyl)-5-prop-1-inyluracil
-1-(β-D-Arabinofuranosyl)-5-prop-2-inyluracil ist,
sowie deren Tautomere und deren physiologisch verträgliche Salze anorganischer und organischer Säuren bzw. Basen, wobei ein aliphatischer Rest in der Definition von R1 und R2 eine Alkyl-, Alkenyl- oder Alkinylgruppe bedeutet.

2. Phospholipid-Derivat nach Anspruch 1, **dadurch gekennzeichnet, daß** Nuc AZT oder FLT ist.

3. Arzneimittel, enthaltend Phospholipid-Derivate der Formel I nach einem der Ansprüche 1 - 2, neben physiologisch verträglichen Hilfs- oder Trägerstoffen.

4. Verwendung von Verbindungen der Formel I nach einem der Ansprüche 1 - 2 zur Herstellung von Arzneimitteln mit antiviraler, antiretroviraler oder antitumoraler Wirkung.

## Claims

1. Phospholipid derivatives of nucleosides of the general formula I 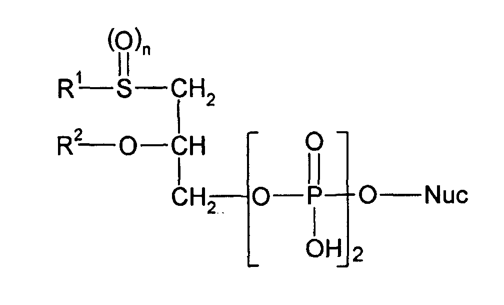 in which
R¹ denotes a straight-chained or branched, saturated or unsaturated aliphatic residue with 9-14 carbon atoms,
R² represents a straight-chained or branched, saturated or unsaturated aliphatic residue with 8-12 carbon atoms
n is 0, 1 or 2,
Nuc can be a nucleoside or a residue derived from the group
-2',3'-dideoxy-3'-azidouridine
-2',3'-dideoxyinosine
-2',3'-dideoxyguanosine
-2',3'-dideoxycytidine
-2',3'-dideoxyadenosine
-2',3'-deoxythymidine
-2',3'-dideoxy-2'-3'-didehydro-N⁶-(o-methylbenzyl)adenosine
-2',3'-dideoxy-2'-3'-didehydro-N⁶-(2-methylpropyl)adenosine
-2',3'-dideoxy-3'-azidoguanosine
-3'-deoxy-3'-azido-thymidine
-2',3'-dideoxy-3'-fluoro-5-chlorouridine
-3'-deoxy-3'-fluorothymidine
-2',3'-dideoxy-3'-fluoroadenosine
-2',3'-dideoxy-3'-fluoro-2,6-diaminopurine-riboside
-2',3'-dideoxy-2'-3'-didehydrocytidine
-3'-deoxy-2'-3'-didehydrothymidine
-5-fluorouridine
-6-mercaptopurine-9-β-D-ribofuranoside
-Acyclovir
-Ganciclovir
-adenine-9-β-D-arabinofuranoside
-2-chloro-2'-deoxyadenosine
-3-(2-deoxy-β-D-erythro-pentofuranosyl)-3,6,7,8-tetrahydroimidazo-[4,5-d][1,3] diazepin-8-ol
-cytosine-9-β-D-arabinofuranoside
-guanine-9-β-D-arabinofuranoside
-hypoxanthine-9-β-D-arabinofuranoside
-2'-deoxy-2-fluoroadenosine
-2-fluoroadenine-9-β-D-arabinofuranoside
-2-fluoroadenosine
-2-amino-6-mercaptopurine-9-β-D-ribofuranoside
-6-methylmercaptopurine-9-β-D-ribofuranoside
-3'-deoxy-5-fluorouridine
-2-chloroadenosine
-3'-deoxy-3'-fluoroadenosine
-3'-deoxy-3'-fluoroguanosine
-1-(β-D-arabinofuranosyl)-5-ethinyluracil
-1-(β-D-arabinofuranosyl)-5-prop-1-inyluracil
-1-(β-D-arabinofuranosyl)-5-prop-2-inyluracil
as well as tautomers thereof and their physiologically tolerated salts of inorganic and organic acids and bases, in which the aliphatic residue in the definiton of R1 and R2 denotes an alkyl, alkenyl or alkinyl group.

2. Phospholipid derivative as claimed in claim 1, wherein Nuc is AZT or FLT.

3. Pharmaceutical preparations containing phospholipid derivatives of formula I as claimed in one of the claims 1-2 in addition to physiologically tolerated auxiliary substances or carriers.

4. Use of compounds of formula I as claimed in one of the claims 1-2 for the production of pharmaceutical agents with antiviral, antiretroviral or antitumoural action.

## Revendications

1. Dérivés phospholipidiques de nucléosides répondant à la formule générale I 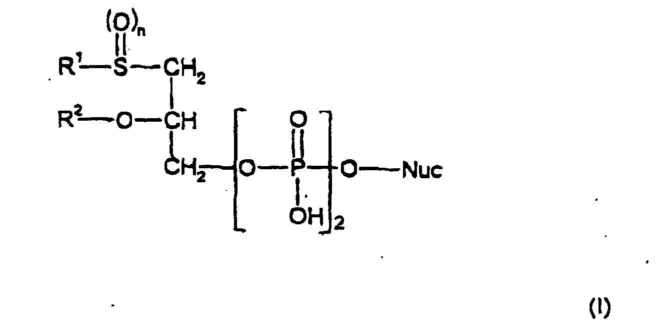 dans laquelle
R¹ représente un radical aliphatique à chaîne droite ou ramifiée, saturé ou insaturé contenant de 9 à 14 atomes de carbone,
R² représente un radical aliphatique à chaîne droite ou ramifiée, saturé ou insaturé contenant de 8 à 12 atomes de carbone,
n est égal à 0, 1 ou 2,
Nuc représente un nucléoside ou un dérivé de nucléoside choisi parmi le groupe constitué de
la 2',3'-didésoxy-3'-azidouridine
la 2',3'-didésoxyinosine
la 2',3'-didésoxyguanosine
la 2',3'-didésoxycytidine
la 2',3'-didésoxyadénosine
la 3'-désoxythymidine
la 2',3'-didésoxy-2',3'-didéhydro-N⁶-(o-méthylbenzyl)adénosine
la 2',3'-didésoxy-2',3'-didéhydro-N⁶-(2-méthylpropyl)adénosine
la 2',3'-didésoxy-3'-azido-guanosine
la 3'-désoxy-3'-azido-thymidine
la 2',3'-didésoxy-3'-fluoro-S-chlorouridine
la 3'-désoxy-3'-fluorothymidine
la 2',3'-didésoxy-3'-fluoroadénosine
la 2',3'-didésoxy-3'-fluoro-2,6-diaminopurineriboside
la 2',3'-didésoxy-2',3'-didéhydrocytidine
la 3'-désoxy-2',3'-didéhydrothymidine
la 5-fluorouridine
le 6-mercaptopurine-9-β-D-ribofuranoside
l'acyclovir
le ganciclovir
l'adénine-9-β-D-arabinofuranoside
la 2-chloro-2'-désoxyadénosine
le 3-(2-désoxy-β-D-érythro-pentofuranosyl)-3,6,7,8-tétrahydro-imidazo-[4,5-d][1,3]diazépin-8-ol
le cytosine-9-β-D-arabinofuranoside
le guanine-9-β-D-arabinofuranoside l'hypoxanthine-9-β-D-arabinofuranoside
le 2'-désoxy-2-fluoroadénosine
le 2-fluoroadénine-9-β-D-arabinofuranoside
la 2-fluoroadénosine
le 2-amino-6-mercaptopurine-9-β-D-ribofuranoside
le 6-méthylmercaptopurine-9-β-D-ribofuranoside
la 3'-désoxy-5-fluorouridine
la 2-chloroadénosine
la 3'-désoxy-3'-fluoroadénosine
la 3'-désoxy-3'-fluoroguanosine
le 1-(β-D-arabinofuranosyl)5-éthynyluracile
le 1-(β-D-arabinofuranosyl)5-prop-1-ynyluracile
le 1-(β-D-arabinofuranosyl)5-prop-2-ynyluracile
ainsi que leurs tautomères et leurs sels physiologiquement acceptables d'acides respectivement de bases inorganiques et organiques, un radical aliphatique dans la définition de R¹ et R² représentant un groupe alkyle, un groupe alcényle ou un groupe alcynyle.

2. Dérivé phospholipidique selon la revendication 1, **caractérisé en ce que** Nuc représente l'AZT ou le FLT.

3. Médicament contenant des dérivés phospholipidiques répondant à la formule I selon l'une quelconque des revendications 1 - 2, à côté d'adjuvants ou de substances de support physiologiquement acceptables.

4. Utilisation de composés répondant à la formule I selon l'une quelconque des revendications 1 - 2 pour la préparation de médicaments à activité antivirale, antirétrovirale ou antitumorale.
